# EUROPEAN PATENT SPECIFICATION

(11) **EP 1 845 867 B1**
(45) Date of publication and mention of the grant of the patent: **21.09.2016**
(21) Application number: 06718164.4
(22) Date of filing: 11.01.2006
(51) Int. Cl.: A61B 17/12

(54) **VASO-OCCLUSIVE DEVICES WITH ATTACHED POLYMER STRUCTURES**
GEFÄSSVERSCHLUSSVORRICHTUNGEN MIT ANGESCHLOSSENEN POLYMERSTRUKTUREN
DISPOSITIFS POUR OCCLUSION VASCULAIRE COMPRENANT DES STRUCTURES POLYMÈRES FIXÉES

(30) Priority: 12.01.2005 US 34893
(43) Date of publication of application: 24.10.2007
(73) Proprietor: Stryker Corporation, Kalamazoo, MI 49002 (US); Stryker European Holdings I, LLC, Kalamazoo, MI 49002 (US)
(72) Inventor: PORTER, Stephen, Christopher, Oakland, California 94618 (US); QUE, Like, Union City, California 94587 (US); DAO, Jimmy, D., Milpitas, California 95035 (US); CASTELLI, Shana, B., San Francisco, California 94117 (US); MURPHY, Richard, Sunnyvale, California 94086 (US); AGANON, Nestor, San Jose, California 95133 (US); CAMINS, Lilibeth, S., Fremont, California 94536 (US)
(74) Representative: Lecomte & Partners
(86) International application number: PCT/US2006/001055
(87) International publication number: WO 2006/076476

(56) References cited:
- WO-A-00/74577
- WO-A-94/09705
- US-A- 5 935 145
- [Online] XP002380195 Retrieved from the Internet: URL:http://gilles.pascale.free.fr/100Assoc iesArchives/Expo/Tissus/Tissus.html> [retrieved on 2003-09-03]

## Description

### FIELD OF THE INVENTION

Compositions and methods for repair of aneurysms are described. In particular, vaso-occlusive devices comprising polymer structures are disclosed, as are methods of making and using these devices.

### BACKGROUND

An aneurysm is a dilation of a blood vessel that poses a risk to health from the potential for rupture, clotting, or dissecting. Rupture of an aneurysm in the brain causes stroke, and rupture of an aneurysm in the abdomen causes shock. Cerebral aneurysms are usually detected in patients as the result of a seizure or hemorrhage and can result in significant morbidity or mortality.

There are a variety of materials and devices which have been used for treatment of aneurysms, including platinum and stainless steel microcoils, polyvinyl alcohol sponges (Ivalone), and other mechanical devices. For example, vaso-occlusion devices are surgical implements or implants that are placed within the vasculature of the human body, typically via a catheter, either to block the flow of blood through a vessel making up that portion of the vasculature through the formation of an embolus or to form such an embolus within an aneurysm stemming from the vessel. One widely used vaso-occlusive device is a helical wire coil having windings that may be dimensioned to engage the walls of the vessels. (*See, e.g.,* U.S. Patent No. 4,994,069 to Ritchart et al.). Electrolytically detachable embolic devices have also been described (U.S. Pat. No. 5,354,295 and its parent, U.S. Pat. No. 5,122,136) as well as vaso-occlusive coils having little or no inherent secondary shape have also been described (*see, e.g.,* co-owned U.S. Patent Numbers 5,690,666; 5,826,587; and 6,458,119).

Coil devices including polymer coatings or attached polymeric filaments have also been described. *See, e.g.,* U.S. Patent No. 5,935,145; 6,033,423; 6,280,457; 6,287,318; and 6,299,627. For instance, U.S. Patent No. 6,280,457 describes wire vaso-occlusive coils having single or multi-filament polymer coatings. U.S. Patent Nos. 6,287,318 and 5,935,145
describe metallic vaso-occlusive devices having a braided polymeric component attached thereto. U.S. Patent No. 5,382,259 describes braids covering a primary coil structure.

Patent application published WO 00/74577 A1 discloses a vaso-occlusive device according to the preamble of claim 1.

However, none of these documents describe vaso-occlusive devices comprising polymeric components as described herein, or methods of making and using such devices.

### SUMMARY OF THE INVENTION

In one aspect, described herein is a vaso-occlusive device according to claim 1. Further developments of the invention are in accordance with dependent claims 1 to 18.

These and other embodiments of the subject invention will readily occur to those of skill in the art in light of the disclosure herein.

### BRIEF DESCRIPTION OF THE FIGURES

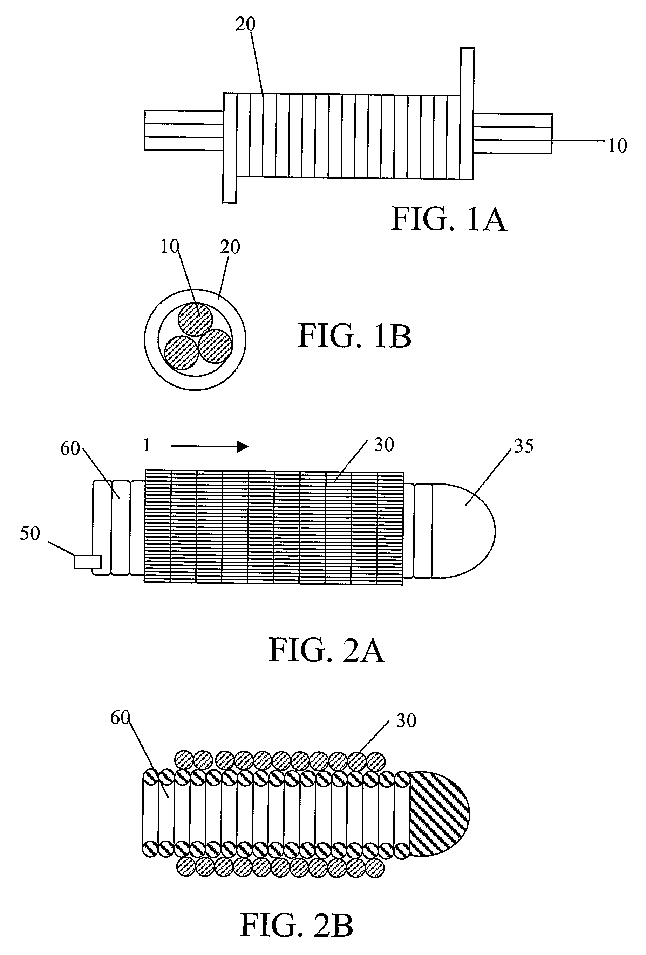

FIG. 1, panels A and B, are side and cross-section views of an exemplary polymeric structure as described herein, in which the polymer component is made by winding at least one outer polymer component around an inner polymer component. FIG. 1A is a side view showing winding of an outer polymer (20) around an inner polymer core (10). FIG. 1B is a cross-section view of the structure shown in FIG. 1A.

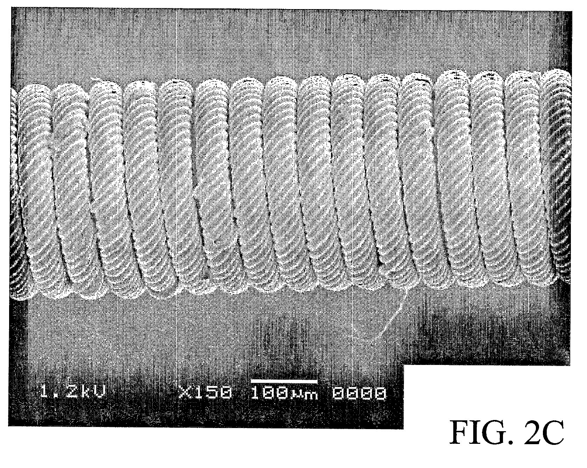

FIG. 2, panels A to C, depict an exemplary multi-layered polymeric structure shown in FIG. 1 wound into a coil (30) then added to a coil-shaped vaso-occlusive device (60). FIG. 2A is a schematic depicting a side view and also shows detachment junction (50). FIG. 2B is a schematic depicting a cross section view. FIG. 2C is a reproduction of a SEM photo showing a side overview of the exemplary multi-layered polymer structure on top of a platinum coil.

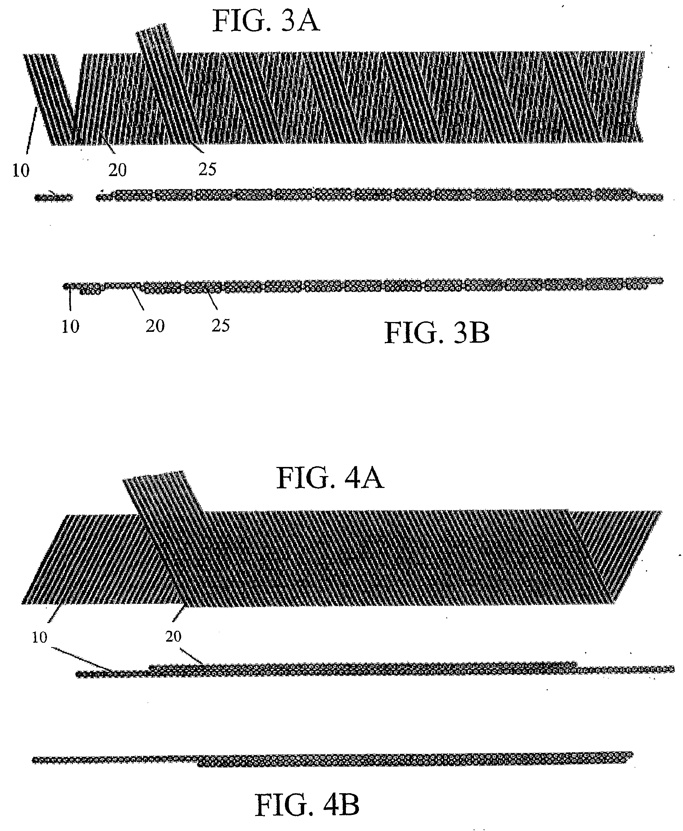

FIG. 3, panels A and B, depict an exemplary multi-layered polymeric structure as described herein that is not part of the invention. In this example, the polymeric component includes 3 layers of flat wound multi-filament yarns. FIG. 3A is a schematic overview showing the inner polymer layer (10) and two outer polymer layers (20, 25). FIG. 3B is a cross-section view of the three-layered polymeric component shown in FIG. 3A.
FIG. 4, panels A and B, depict another exemplary multi-layered polymer as described herein. In this example, the polymeric component includes 2 layers of flat wound multi-filament yarns. FIG. 4A is a schematic overview showing the inner polymer layer (10) and outer polymer layer (20). FIG. 4B is a cross-section view of the three-layered polymer shown in FIG. 4A.

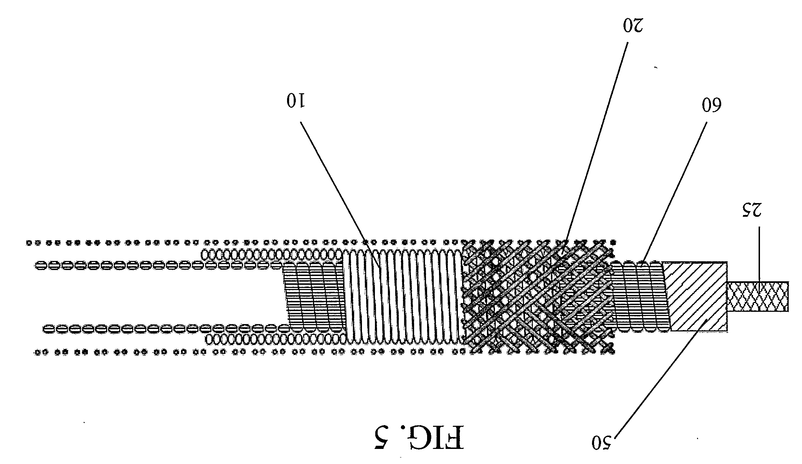

FIG. 5 is a partial side-view, partial cross-section view of an exemplary device as described herein. In this example, the inner polymer member (10) comprises a closed pitch coil and the outer polymer member (20) comprises a braided tubular structure.

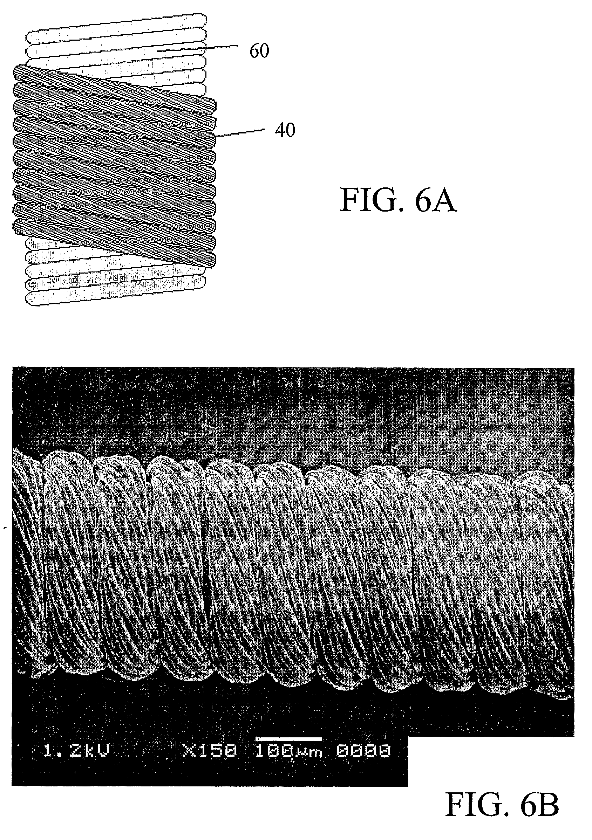

FIG. 6, panels A and B, depict an exemplary twisted polymeric construction (40) on top of a coil-shaped vaso-occlusive device (60). FIG. 6A is a schematic depicting a twisted polymer structure over a coil. FIG. 6B is a reproduction of is a reproduction of a SEM photo showing a side overview of an exemplary twisted polymeric component on top of a platinum coil.

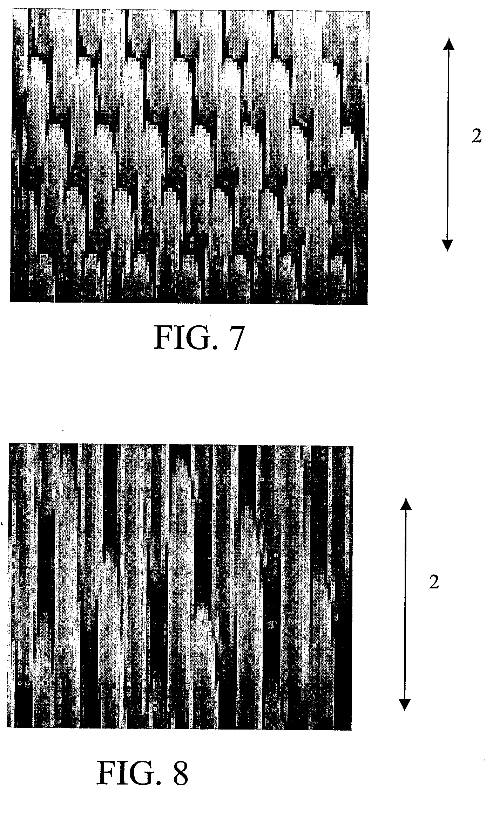

FIG. 7 is a magnified view of a twill-woven polymer structure.
FIG. 8 is a magnified view of a satin-woven polymer structure.

### DESCRIPTION OF THE INVENTION

Occlusive (*e.g*., embolic) compositions are described. The compositions described herein find use in vascular and neurovascular indications and are particularly useful in treating aneurysms, for example small-diameter, curved or otherwise difficult to access vasculature, for example aneurysms, such as cerebral aneurysms. The compositions and methods described herein may achieve better occlusion and treatment outcomes than known devices, for example because they can be deployed with less friction and/or achieve higher packing densities.

Advantages of the present invention include, but are not limited to, (i) the provision of low-friction polymer covered vaso-occlusive devices; (ii) the provision of occlusive elements that can be packed into aneurysms at high densities; (iv) the provision of occlusive devices that can be retrieved and/or repositioned after deployment; and (v) cost-effective production of these devices.

It must be noted that, as used in this specification and the appended claims, the singular forms "a", "an", and "the" include plural referents unless the content clearly dictates otherwise. Thus, for example, reference to a device comprising "a polymer" includes devices comprising of two or more polymers.

The novel vaso-occlusive elements described herein comprise at least one polymer structure made up of two or more polymer filaments, for example constructs comprising filamentous elements assembled by one or more operations including coiling, twisting, braiding, weaving or knitting of the filamentous elements. Thus, non-limiting examples of polymer structures include multi-layered polymers, twisted polymer constructions, twill woven polymers, and satin woven polymers.

The polymer(s) making up the structures described herein may be selected from a wide variety of materials. One such example is a suture-type material. Synthetic and natural polymers, such as polyurethanes (including block copolymers with soft segments containing esters, ethers and carbonates), polyethers, polyamides (including nylon polymers and their derivatives), polyimides (including both thermosetting and thermoplastic materials), acrylates (including cyanoacrylates), epoxy adhesive materials (two part or one part epoxy-amine materials), olefins (including polymers and copolymers of ethylene, propylene butadiene, styrene, and thermoplastic olefin elastomers), fluoronated polymers (including polytetrafluoroethylene), polydimethyl siloxane-based polymers, cross-linked polymers, non-cross linked polymers, Rayon, cellulose, cellulose derivatives such nitrocellulose, natural rubbers, polyesters such as lactides, glycolides, trimethylene carbonate, caprolactone polymers and their copolymers, hydroxybutyrate and polyhydroxyvalerate and their copolymers, polyether esters such as polydioxinone, anhydrides such as polymers and copolymers of sebacic acid, hexadecandioic acid and other diacids, or orthoesters may be used.

Thus, the polymer structures described herein may include one or more absorbable (biodegradable) polymers and/or one or more non-absorbable polymers. The terms "absorbable" and "biodegradable" are used interchangeable to refer to any agent that, over time, is no longer identifiable at the site of application in the form it was injected, for example having been removed via degradation, metabolism, dissolving or any passive or active removal procedure. Non-limiting examples of absorbable proteins include synthetic and polysaccharide biodegradable hydrogels, collagen, elastin, fibrinogen, fibronectin, vitronectin, laminin and gelatin. Many of these materials are commercially available. Fibrin-containing compositions are commercially available, for example from Baxter. Collagen containing compositions are commercially available, for example from Cohesion Technologies, Inc., Palo Alto, California. Fibrinogen-containing compositions are described, for example, in U.S. Patent Nos. 6,168,788 and 5,290,552. Mixtures, copolymers (both block and random) of these materials are also suitable.

Preferred biodegradable polymers include materials used as dissolvable suture materials, for instance polyglycolic and polylactic acids to encourage cell growth in the aneurysm after their introduction. Preferred non-biodegradable polymers include polyethylene teraphthalate (PET or Dacron), polypropylene, polytetraflouroethylene, or Nylon materials. Highly preferred is PET, for instance, in the form of 10-0 and 9-0 PET suture material or other small diameter multifilament yarns.

In addition to the polymeric component, the devices described herein also typically include a core element. The core element may be made of a variety of materials (*e.g.*, metal, polymer, etc.) and may assume a variety of tubular structures, for examples, braids, coils, combination braid and coils and the like. Similarly, although depicted in the Figures described below as a coil, the inner member may be of a variety of shapes or configuration includes, but not limited to, braids, knits, woven structures, tubes (*e.g*., perforated or slotted tubes), cables, injection-molded devices and the like. *See, e.g.,* U.S. Patent No. 6,533,801 and International Patent Publication WO 02/096273. The core element preferably changes shape upon deployment, for example change from a constrained linear form to a relaxed, three-dimensional (secondary) configuration. *See, also,* U.S. Patent No. 6,280,457.

In a particularly preferred embodiment, the core element comprises at least one metal or alloy. Suitable metals and alloys for the core element include the Platinum Group metals, especially platinum, rhodium, palladium, rhenium, as well as tungsten, gold, silver, tantalum, and alloys of these metals. The core element may also comprise of any of a wide variety of stainless steels if some sacrifice of radio-opacity may be tolerated. Very desirable materials of construction, from a mechanical point of view, are materials that maintain their shape despite being subjected to high stress. Certain "super-elastic alloys" include nickel/titanium alloys (48-58 atomic % nickel and optionally containing modest amounts of iron); copper/zinc alloys (38-42 weight % zinc); copper/zinc alloys containing 1-10 weight % of beryllium, silicon, tin, aluminum, or gallium; or nickel/aluminum alloys (36-38 atomic % aluminum). Particularly preferred are the alloys described in U.S. Pat. Nos. 3,174,851; 3,351,463; and 3,753,700. Especially preferred is the titanium/nickel alloy known as "nitinol," These are very sturdy alloys that will tolerate significant flexing without deformation even when used as a very small diameter wire. If a super-elastic alloy such as nitinol is used in any component of the device, the diameter of the wire may be significantly smaller than that used when the relatively more ductile platinum or platinum/tungsten alloy is used as the material of construction. These metals have significant radio-opacity and in their alloys may be tailored to accomplish an appropriate blend of flexibility and stiffness. They are also largely biologically inert. In a preferred embodiment, the core element comprises a metal wire wound into a primary helical shape. The core element may be, but is not necessarily, subjected to a heating step to set the wire into the primary shape. The diameter of the wire typically making up the coils is often in a range of 0.0127 mm (0.005 inches) and 1.27 mm (0.050 inches) preferably between about 0.0254 mm (0.001 inches) and about 0.1016 mm (0.004 inches) in diameter.

As shown in the Figures, the polymeric structures described herein preferably surround most, or all, of the surface of the core element and may be combined with the core element in any fashion. For example, the polymeric structures may be wound around the core element or, alternatively, may be shaped into a tubular sheath that surrounds the core element. The polymer component may adhere to the core element in one or more locations, for example by heating of the polymer or by use of adhesives (*e.g.,* EVA) to the polymer or to the core element) or by other suitable means. Furthermore, the polymeric component may be added to the core element before or after the core element is shaped into a primary and/or secondary configuration. The use of the polymer structures as described herein on known vaso-occlusive devices (core elements) results in much less friction upon delivery and/or deployment and, in addition, allows for higher density packing of vaso-occlusive coils into vessels (*e.g.*, aneurysms).

FIGs. 1, panels A and B, show an exemplary polymeric construction as described herein in side and cross section views. In this embodiment, outer polymer member (20) is helically wound around inner polymer member (10). Preferably, in this embodiment, the outer polymer member (20) is wound around inner polymer member (10) with a closed pitch such that the inner polymer member is not exposed but is completely covered by the outer polymer member.

The resulting inner (10) and outer (20, 25) member wound construction can then be wound into another shape, for example a helically shaped coil, and optionally heat treated so as to retain the secondary shape. FIGs. 2A and 2B show the multi-layered polymer of FIG. 1A and 1B which has been wound into a coil configuration (30) in combination with a coil shaped core element (60). Also shown in FIG. 2A is detachment junction (50) and optional tip (35) to ease the potential of the component wire to cause trauma in a blood vessel. The orientation of the outer polymer member (20) of the multi-layered polymer (30) is preferably parallel (arrow 1) to the direction of coil travel (arrow 1), thereby reducing coil-on-coil friction during deployment.

FIG. 3 shows an exemplary multi-layered polymer structure comprising 3 layers of multifilament yarn polymers. As shown in the embodiment of FIG. 3A, inner layer (10) is wound with open pitch. In this example, the second layer, outer layer (20) is preferably wound with a closed pitch. Furthermore, as depicted in FIG. 3A second layer (20) is preferably oriented (wound) in a different direction than inner layer (10). Third layer, outer layer (25) is preferably wound with an open pitch (*e.g*., same pitch as inner layer (10). Preferably, third layer outer member (25) is offset as compared to inner layer (10), for example by ½ pitch length. In addition, it is preferred that each layer of yarn be flat wound.

FIG. 4 shows another exemplary multi-layered polymer structure similar to that shown in FIGs. 2 and 3. This example comprises 2 layers of multifilament yarns. As with the embodiment shown in FIG. 3, the yarn of each layer is preferably flat wound. Inner (10) and outer (20) polymer may be wound with an open or closed pitch, although it may be preferable to wind outer member (20) is a closed pitch. Preferably, outer member (20) in this embodiment is wound in a different direction than inner member (10).

FIG. 5 shows another exemplary multi-layered polymeric structure in which the outer (20) polymer is a tubular braided structure and the inner (10) polymer member is wound around the core element in a closed pitch. Also shown are pusher wire (25) and detachment junction (50). The inclusion of a tubular braided structure may help reduce friction and ease delivery of the device.

Other multi-layered polymer configurations having from 2-8 layers comprising a combination of open and/or closed pitch coil constructions are also possible.

Inner (10) and/or outer (20) polymer members may be any polymer or combination of polymers, for example as described above. Furthermore, inner (10) and/or outer members (20) may comprise a braided polymer, a single polymer monofilament and/or multi-filaments (*e.g.*, multifilament yarns, threads or sutures).

In certain embodiments, inner and outer members comprise at least one biodegradable polymer. In other embodiments, inner polymer member (10) comprises at least one biodegradable polymer and outer polymer member (20) comprises non-biodegradable polymers (*e.g.,* Nylon). In still other embodiments, inner polymer member (10) comprises one or more non-biodegradable polymers and outer polymer member (20) comprises non-biodegradable polymers. The use of a non-biodegradable outer member (20) with a larger mesh than the inner member (10) may help prevent the premature degradation (*e.g.,* release of particles) from an inner member (10) that includes biodegradable polymer(s). For instance if the inner member (10) comprises an absorbable polymer *(e.g.,* braided suture) and the outer member (20) comprises an non-absorbable polymer (*e.g.,* Nylon filament) having a slightly larger mesh or pitch than the absorbable polymer, particulate matter that is larger than the mesh size of the non-absorbable polymer cannot be released during deployment. This design does, however, allow appropriate degradation of the inner polymer (*e.g.*, through the openings in the non-absorbable polymer) after deployment.

As noted above, the polymer structures described herein may be made to adhere to the underlying wire/coil (60) by melting the polymer(s) (*e.g.*, outer polymer) or by the use of adhesives (*e.g*., by addition of adhesives such as ethylvinylacetate (EVA) to the polymer component or to the core element) or by other suitable means. In these embodiments, the secured polymer covered wire can then be wound into a helical shape.

Alternatively, the polymeric coil structure (30) can be added to an already wound helically shaped coil, for example by loading the polymeric coil onto an underlying coil, securing the polymeric coil to the underlying coil at one or more locations (*e.g*., by using ultraviolet glue to fix the ends of the multi-layered polymeric coil to the underlying coil and optionally heat setting the device so shrink the multi-layered polymeric coil to the underlying coil. The polymer component may completely cover the core element (as shown in FIG. 2C) or may be added to the core element such that one or more regions of the core element are not covered.

In still other embodiments, the polymeric structure comprises a twisted fiber structure. Unlike existing polymer coverings, which use braided multifilaments or monofilaments, the twisted polymers described herein comprise yarns, filaments or fibers that are twisted into a tight cable-like structure. The amount of twist, direction of twist and composition of the polymer fibers may be varied to promote greater regularity and/or stability. The twisted cable-like structures can then be wound into another shape, for example a helically shaped coil, and optionally heat-treated so as to retain the secondary shape.

FIG. 6A is a schematic depiction of a twisted polymer structure (40) added to an underlying coil (60). FIG. 6B shows an exemplary twisted polymer component covering a platinum coil. The twisted polymer may entirely (FIG. 6B) or partially cover the underlying core element.

In preferred embodiments, a twisted polymer as described herein comprises between about 5 and 100 (and any integer therebetween), more preferably between about 6 and 50 (or any integer therebetween) and even more preferably between about 7 and 20 (or any integer therebetween) individual filaments that are used for forming the twisted polymer structure. Although the diameter of the fibers and filaments may vary greatly, the preferred diameter of the twisted fibers is between about 0.0381 mm (0.0015 inches) and about 0.0508 mm (0.0020 inches) and the preferred diameter of the filaments is between about 0.00762 mm (0.0003 inches) and about 0.02032 mm (0.0008 inches). In addition, although the amount of twist may vary greatly, it is preferred that the twisted structures have between about 10 and about 100 turns per inch. Furthermore, when used in coiled tube geometry, the direction of twist is preferably opposite to the direction of the coil, for instance if the polymer is formed into a coil having turns in "Z" direction, the twisted polymer has turns in an "S" direction.

Any of the aforementioned polymeric structures may be covered with a braided tubular outer *(e.g.,* outer member (20) of FIG. 5). Such tubular structures may further enhance the low-friction properties of the embolic device.

In other embodiments, the polymer component comprises a twill weave or satin weave pattern. The term "weave" refers generally to the pattern created by the weaving of warp (vertical) fibers with weft (horizontal) fibers. The twill weave shown in FIG. 7 is a weave construction in which one or more warp fibers run over two or more weft fibers, resulting in a weave in which individual fibers on one surface of the weave are parallel. The nature of the twill weave construction provides smaller mesh openings than the conventional over-under weaves and, in addition, provides increased smoothness and drapeability (over contoured surfaces). Preferably, a twill weave polymer as shown in FIG. 7 is used as a sheath for a coil-shaped core element. Furthermore, as shown by arrow (2) in FIG. 7, the twill weave is preferably oriented on the core element so that the individual fibers on the outer surface of the sheath are parallel to the long axis of the coiled core element.

FIG. 8 shows another example in which the polymeric structure comprises a satin weave. The satin weave shown in FIG. 8 is a weave construction in which one warp fiber floats over three or more weft fibers, arranged such that the surface is compact with no distinguishable twill line. The decreased interlacing between warp and weft fibers of a satin weave, and resulting increase in distance between support fibers, reduces the flexural modulus in the principle fiber direction. In other words, a satin weave configuration conforms easily around most contoured surfaces and indeed, is considered to be one of the most drapeable weave patterns. Accordingly, a satin weave polymer as shown in FIG. 8 is preferably configured to form a sheath that surrounds a coil-shaped core element. Furthermore, as shown by arrow (2) in FIG. 8, the satin weave structure is preferably oriented so that the principle fiber direction is on the outer surface of the sheath and parallel to the long axis of the core element.

As noted above, the polymer structures described herein are advantageously used in combination with a core element, for example a platinum coil. Methods of making vaso-occlusive coils having a linear helical shape and/or a different three-dimensional (secondary) configuration are known in the art and described in detail in the documents cited above, for example in U.S. Patent No. 6,280,457. Thus, it is further within the scope of this invention that the vaso-occlusive device as a whole or elements thereof comprise secondary shapes or structures that differ from the linear coil shapes depicted in the Figures, for examples, spheres, ellipses, spirals, ovoids, figure-8 shapes, etc. The devices described herein may be self-forming in that they assume the secondary configuration upon deployment into an aneurysm. Alternatively, the devices may assume their secondary configurations under certain conditions (*e.g*., change in temperature, application of energy, etc.). Stretch-resistant configurations can also be designed and manufactured. For example, a fiber material can be threaded through the inside of the core element and secured to both the proximal and distal end of the device. *See, e.g.,* U.S. Patent No. 6,280,457.

One or more of the polymer structures described herein and core elements may also comprise additional components (described in further detail below), such as co-solvents, plasticizers, radio-opaque materials (*e.g.*, metals such as tantalum, gold or platinum), coalescing solvents, bioactive agents, antimicrobial agents, antithrombogenic agents, antibiotics, pigments, radiopacifiers and/or ion conductors which may be coated using any suitable method or may be incorporated into the element(s) during production. In addition, lubricious materials (*e.g.*, hydrophilic) materials may be used to coat one or more members of the device to help facilitate delivery. Cyanoacrylate resins (particularly n-butylcyanoacrylate), particular embolization materials such as microparticles of polyvinyl alcohol foam may also be introduced into the intended site after the inventive devices are in place. Furthermore, previously described fibrous braided and woven components (U.S. Patent No. 5,522,822) may also be included, for example surrounding the polymeric structure-covered core elements described herein.

One or more bioactive materials may also be included. *See, e.g.,* co-owned U.S. Patent No. 6,585,754 and WO 02/051460. The term "bioactive" refers to any agent that exhibits effects *in vivo,* for example a thrombotic agent, an anti-thrombotic agent *(e.g.,* a water-soluble agent that inhibits thrombosis for a limited time period, described above), a therapeutic agent (*e.g.*, chemotherapeutic agent) or the like. Non-limiting examples of bioactive materials include cytokines; extracellular matrix molecules (*e.g.*, collagen); trace metals (*e.g.*, copper); and other molecules that stabilize thrombus formation or inhibit clot lysis (*e.g*., proteins or functional fragments of proteins, including but not limited to Factor XIII, α₂-antiplasmin, plasminogen activator inhibitor-1 (PAI-1) or the like). Non-limiting examples of cytokines which may be used alone or in combination in the practice of the present invention include, basic fibroblast growth factor (bFGF), platelet derived growth factor (PDGF), vascular endothelial growth factor (VEGF), transforming growth factor beta (TGF-β) and the like. Cytokines, extracellular matrix molecules and thrombus stabilizing molecules (*e.g.*, Factor XIII, PAI-1, etc.) are commercially available from several vendors such as, for example, Genzyme (Framingham, MA), Genentech (South San Francisco, CA), Amgen (Thousand Oaks, CA), R&D Systems and Immunex (Seattle, WA). Additionally, bioactive polypeptides can be synthesized recombinantly as the sequences of many of these molecules are also available, for example, from the GenBank database. Thus, it is intended that the invention include use of DNA or RNA encoding any of the bioactive molecules. Cells (*e.g*., fibroblasts, stem cells, etc.) can also be included. Such cells may be genetically modified. Furthermore, it is intended, although not always explicitly stated, that molecules having similar biological activity as wild-type or purified cytokines, extracellular matrix molecules and thrombus-stabilizing proteins (e.g., recombinantly produced or mutants thereof) and nucleic acid encoding these molecules are intended to be used within the spirit and scope of the invention. Further, the amount and concentration of liquid embolic and/or other bioactive materials useful in the practice of the invention can be readily determined by a skilled operator and it will be understood that any combination of materials, concentration or dosage can be used, so long as it is not harmful to the subject.

The devices described herein are often introduced into a selected site using the procedure outlined below. This procedure may be used in treating a variety of maladies. For instance in the treatment of an aneurysm, the aneurysm itself will be filled (partially or fully) with the compositions described herein.

Conventional catheter insertion and navigational techniques involving guidewires or flow-directed devices may be used to access the site with a catheter. The mechanism will be such as to be capable of being advanced entirely through the catheter to place vaso-occlusive device at the target site but yet with a sufficient portion of the distal end of the delivery mechanism protruding from the distal end of the catheter to enable detachment of the implantable vaso-occlusive device. For use in peripheral or neural surgeries, the delivery mechanism will normally be about 100-200 cm in length, more normally 130-180 cm in length. The diameter of the delivery mechanism is usually in the range of 0.25 to about 0.90 mm. Briefly, occlusive devices (and/or additional components) described herein are typically loaded into a carrier for introduction into the delivery catheter and introduced to the chosen site using the procedure outlined below. This procedure maybe used in treating a variety of maladies. For instance, in treatment of an aneurysm, the aneurysm itself may be filled with the embolics (*e.g.* vaso-occlusive members and/or liquid embolics and bioactive materials) which cause formation of an emboli and, at some later time, is at least partially replaced by neovascularized collagenous material formed around the implanted vaso-occlusive devices.

A selected site is reached through the vascular system using a collection of specifically chosen catheters and/or guide wires. It is clear that should the site be in a remote site, e.g., in the brain, methods of reaching this site are somewhat limited. One widely accepted procedure is found in U.S. Patent No. 4,994,069 to Ritchart, et al. It utilizes a fine endovascular catheter such as is found in U.S. Patent No. 4,739,768, to Engelson. First of all, a large catheter is introduced through an entry site in the vasculature. Typically, this would be through a femoral artery in the groin. Other entry sites sometimes chosen are found in the neck and are in general well known by physicians who practice this type of medicine. Once the introducer is in place, a guiding catheter is then used to provide a safe passageway from the entry site to a region near the site to be treated. For instance, in treating a site in the human brain, a guiding catheter would be chosen which would extend from the entry site at the femoral artery, up through the large arteries extending to the heart, around the heart through the aortic arch, and downstream through one of the arteries extending from the upper side of the aorta. A guidewire and neurovascular catheter such as that described in the Engelson patent are then placed through the guiding catheter. Once the distal end of the catheter is positioned at the site, often by locating its distal end through the use of radiopaque marker material and fluoroscopy, the catheter is cleared. For instance, if a guidewire has been used to position the catheter, it is withdrawn from the catheter and then the assembly, for example including the absorbable vaso-occlusive device at the distal end, is advanced through the catheter.

Once the selected site has been reached, the vaso-occlusive device is extruded, for example by loading onto a pusher wire. Preferably, the vaso-occlusive device is loaded onto the pusher wire via a mechanically or electrolytically cleavable junction (*e.g.*, a GDC-type junction that can be severed by application of heat, electrolysis, electrodynamic activation or other means). Additionally, the vaso-occlusive device can be designed to include multiple detachment points, as described in co-owned U.S. Patent No. 6,623,493 and 6,533,801 and International Patent publication WO 02/45596. They are held in place by gravity, shape, size, volume, magnetic field or combinations thereof.

Modifications of the procedure and vaso-occlusive devices described above, in keeping with this invention will be apparent to those having skill in this mechanical and surgical art. These variations are intended to be within the scope of the claims that follow.

## Claims

1. A vaso-occlusive device comprising a core element (60) having an outer surface and at least one polymer structure surrounding the surface of the core element, **characterized in that** the polymer structure comprises an outer polymer member (20) helically wound around an inner polymer member (10), and the resulting wound construction is wound around the core element (60).

2. The vaso-occlusive device of claim 1, wherein the outer polymer member (20) is wound around the inner polymer member (10) with a closed pitch.

3. The vaso-occlusive device of any of claim 1 or 2, wherein one of the inner polymer member (10) or the outer polymer member (20) is formed of a biodegradable polymer and the other of the inner polymer member (10) or the outer polymer member (20) is formed of a non-biodegradable polymer.

4. The vaso-occlusive device of claim 3, wherein the biodegradable polymers is comprised of at least one polymer selected form group consisting of: lactide, glycolide, trimethylene carbonate and caprolactone polymers and their copolymers; hydroxybutyrate and polyhydroxyvalerate and their block and random copolymers; a polyether ester; anhydrides, polymers and copolymers of sebacic acid, hexadecandioic acid; and orthoesters.

5. The vaso-occlusive device of claim 3 or 4, wherein the non-biodegradable polymer is selected from the group consisting of polyethylene teraphthalate, polytetraflouroethylene, polyurethane, polypropylene and Nylon materials.

6. The vaso-occlusive device of claims 1 to 5, wherein the inner polymer member (10) is comprised of monofilaments or multi-filaments.

7. The vaso-occlusive device of claim 6, wherein the inner polymer member (10) is wound into a helical shape.

8. The vaso-occlusive device of claim 6 or 7, wherein the inner polymer member (10) and the outer polymer member (20) are wound into a helical shape.

9. The vaso-occlusive device of claims 6 to 8, wherein the inner polymer member (10) comprises a multi-filament yarn and the outer polymer member (20) comprises a multi-filament yarn.

10. The vaso-occlusive device of claims 6 to 9, wherein the inner polymer member (10) comprises a closed pitch helical coil and the outer polymer member (20) comprises a braided tubular structure.

11. The vaso-occlusive device of claims 1 to 10, wherein the core element comprises a wire formed into a helically wound primary shape.

12. The vaso-occlusive device of claim 11, where the core element has a secondary shape that self-forms upon deployment.

13. The vaso-occlusive device of claim 12, where the secondary shape is selected from the group consisting of cloverleaf shaped, helically-shaped, figure-8 shaped, flower-shaped, vortex-shaped, ovoid, randomly shaped, and substantially spherical.

14. The vaso-occlusive device of claim 13, wherein the secondary shape comprises a series of conjoined helical segments.

15. The vaso-occlusive device of claim 14, wherein at least two of the conjoined helical segments have differing diameters or differing orientation axes.

16. The vaso-occlusive device of any of claims 1 to 15, wherein the device is radioopaque.

17. The vaso-occlusive device of any of claims 1 to 16, further comprising a detachment junction.

18. The vaso-occlusive device of claim 17, wherein the detachable junction is electrolytically detachable.

## Patentansprüche

1. Gefäßverschlussvorrichtung, umfassend ein Kernelement (60) mit einer äußeren Oberfläche und mindestens einer Polymerstruktur, welche die Oberfläche des Kernelements umgibt, **dadurch gekennzeichnet, dass** die Polymerstruktur ein äußeres Polymerelement (20) umfasst, das schraubenförmig um ein inneres Polymerelement (10) herumgewickelt ist, und die resultierende gewickelte Konstruktion um das Kernelement (60) herum gewickelt ist.

2. Gefäßverschlussvorrichtung nach Anspruch 1, wobei das äußere Polymerelement (20) mit einer geschlossenen Windungssteigung um das innere Polymerelement (10) herumgewickelt ist.

3. Gefäßverschlussvorrichtung nach einem von Anspruch 1 oder 2, wobei eines des inneren Polymerelements (10) oder des äußeren Polymerelements (20) aus einem biologisch abbaubaren Polymer gebildet ist und das andere des inneren Polymerelements (10) oder des äußeren Polymerelements (20) aus einem nicht biologisch abbaubaren Polymer gebildet ist.

4. Gefäßverschlussvorrichtung nach Anspruch 3, wobei das biologisch abbaubare Polymer aus mindestens einem Polymer besteht, ausgewählt aus der Gruppe, bestehend aus: Lactid, Glycolid, Trimethylencarbonat und Caprolacton-Polymeren und deren Copolymeren; Hydroxybutyrat und Polyhydroxyvalerat und deren Block- und statistischen Copolymeren; einem Polyetherester; Anhydriden, Polymeren und Copolymeren von Sebacinsäure, Hexadecandisäure; und Orthoestern.

5. Gefäßverschlussvorrichtung nach Anspruch 3 oder 4, wobei das nicht biologisch abbaubare Polymer aus der Gruppe ausgewählt ist, bestehend aus Polyethylenterephthalat, Polytetrafluorethylen, Polyurethan, Polypropylen und Nylonmaterialien.

6. Gefäßverschlussvorrichtung nach Ansprüchen 1 bis 5, wobei das innere Polymerelement (10) aus Monofilamenten oder Multifilamenten besteht.

7. Gefäßverschlussvorrichtung nach Anspruch 6, wobei das innere Polymerelement (10) zu einer schraubenförmigen Form gewickelt ist.

8. Gefäßverschlussvorrichtung nach Anspruch 6 oder 7, wobei das innere Polymerelement (10) und das äußere Polymerelement (20) zu einer schraubenförmigen Form gewickelt sind.

9. Gefäßverschlussvorrichtung nach Ansprüchen 6 bis 8, wobei das innere Polymerelement (10) ein Multifilamentgarn umfasst und das äußere Polymerelement (20) ein Multifilamentgarn umfasst.

10. Gefäßverschlussvorrichtung nach Ansprüchen 6 bis 9, wobei das innere Polymerelement (10) eine schraubenförmige Wendel mit geschlossener Windungssteigung umfasst und das äußere Polymerelement (20) eine geflochtene röhrenförmige Struktur umfasst.

11. Gefäßverschlussvorrichtung nach Ansprüchen 1 bis 10, wobei das Kernelement einen Draht umfasst, der zu einer schraubenförmig gewickelten primären Form geformt ist.

12. Gefäßverschlussvorrichtung nach Anspruch 11, wobei das Kernelement eine sekundäre Form aufweist, die sich nach dem Einsetzen selbst formt.

13. Gefäßverschlussvorrichtung nach Anspruch 12, wobei die sekundäre Form aus der aus kleeblattförmig, schraubenförmig, Ziffer-8-förmig, blütenförmig, spiralförmig, eiförmig, willkürlich geformt, und im Wesentlichen kugelförmig bestehenden Gruppe ausgewählt ist.

14. Gefäßverschlussvorrichtung nach Anspruch 13, wobei die sekundäre Form eine Serie von miteinander verbundenen schraubenförmigen Segmenten umfasst.

15. Gefäßverschlussvorrichtung nach Anspruch 14, wobei mindestens zwei der miteinander verbundenen schraubenförmigen Segmente unterschiedliche Durchmesser oder unterschiedliche Orientierungsachsen aufweisen.

16. Gefäßverschlussvorrichtung nach einem der Ansprüche 1 bis 15, wobei die Vorrichtung strahlungsundurchlässig ist.

17. Gefäßverschlussvorrichtung nach einem der Ansprüche 1 bis 16, weiter eine Ablösungs-Verbindungsstelle umfassend.

18. Gefäßverschlussvorrichtung nach Anspruch 17, wobei die lösbare Verbindungsstelle elektrolytisch lösbar ist.

## Revendications

1. Dispositif pour l'occlusion vasculaire comprenant un élément central (60) possédant une surface externe et au moins une structure polymère entourant la surface de l'élément central, **caractérisé en ce que** la structure polymère comprend un élément polymère externe (20) qui vient s'enrouler de manière hélicoïdale autour d'un élément polymère interne (10) et la structure enroulée résultante vient s'enrouler autour de l'élément central (60).

2. Dispositif pour l'occlusion vasculaire selon la revendication 1, dans lequel l'élément polymère externe (20) vient s'enrouler autour de l'élément polymère interne (10) avec un pas serré.

3. Dispositif pour l'occlusion vasculaire selon l'une quelconque des revendications 1 ou 2, dans lequel un élément choisi parmi l'élément polymère interne (10) et l'élément polymère externe (20) est constitué d'un polymère biodégradable et l'autre élément choisi parmi l'élément polymère interne (10) et l'élément polymère externe (20) est constitué d'un polymère non biodégradable.

4. Dispositif pour l'occlusion vasculaire selon la revendication 3, dans lequel le polymère biodégradable comprend au moins un polymère choisi parmi le groupe constitué par : des polymères de lactide, de glycolide, de carbonate de triméthylène et de caprolactone, et leurs copolymères ; l'hydroxybutyrate et le polyhydroxyvalérate, ainsi que leurs copolymères séquencés et statistiques ; un polyéther-ester ; des anhydrides, des polymères et des copolymères de l'acide sébacique, de l'acide hexadécanedioïque ; et des orthoesters.

5. Dispositif pour l'occlusion vasculaire selon la revendication 3 ou 4, dans lequel le polymère non biodégradable est choisi parmi le groupe constitué par le polyéthylène téréphtalate, le polytétrafluoréthylène, le polyuréthane, le polypropylène et des matières à base de nylon.

6. Dispositif pour l'occlusion vasculaire selon les revendications 1 à 5, dans lequel l'élément polymère interne (10) comprend des monofilaments ou des multifilaments.

7. Dispositif pour l'occlusion vasculaire selon la revendication 6, dans lequel l'élément polymère interne (10) s'enroule pour prendre une configuration de forme hélicoïdale.

8. Dispositif pour l'occlusion vasculaire selon la revendication 6 ou 7, dans lequel l'élément polymère interne (10) et l'élément polymère externe (20) s'enroulent pour prendre une configuration de forme hélicoïdale.

9. Dispositif pour l'occlusion vasculaire selon les revendications 6 à 8, dans lequel l'élément polymère interne (10) comprend un fil à multifilaments et l'élément polymère externe (20) comprend un fil à multifilaments.

10. Dispositif pour l'occlusion vasculaire selon les revendications 6 à 9, dans lequel l'élément polymère interne (10) comprend un serpentin hélicoïdal à pas serré et l'élément polymère externe (20) comprend une structure tubulaire tressée.

11. Dispositif pour l'occlusion vasculaire selon les revendications 1 à 10, dans lequel l'élément central comprend un fil métallique qui prend une configuration primaire sous la forme d'un enroulement hélicoïdal.

12. Dispositif pour l'occlusion vasculaire selon la revendication 11, dans lequel l'élément central possède une configuration secondaire qui se met en place de manière automatique lors du déploiement du premier cité.

13. Dispositif pour l'occlusion vasculaire selon la revendication 12, dans lequel la configuration secondaire est choisie parmi le groupe constitué par une configuration en forme de feuille de trèfle, une configuration de forme hélicoïdale, une configuration en forme de chiffre 8, une configuration en forme de fleur, une configuration en forme de spirale, une configuration de forme ovoïde, une configuration de forme aléatoire et une configuration de forme essentiellement sphérique.

14. Dispositif pour l'occlusion vasculaire selon la revendication 13, dans lequel la configuration secondaire comprend une série de segments hélicoïdaux joints les uns aux autres.

15. Dispositif pour l'occlusion vasculaire selon la revendication 14, dans lequel au moins deux des segments hélicoïdaux joints les uns aux autres possèdent des diamètres qui diffèrent ou des axes d'orientation qui diffèrent.

16. Dispositif pour l'occlusion vasculaire selon les revendications 1 à 15, dans lequel le dispositif est opaque aux rayonnements.

17. Dispositif pour l'occlusion vasculaire selon les revendications 1 à 16, comprenant en outre un joint détachable.

18. Dispositif pour l'occlusion vasculaire selon la revendication 17, dans lequel le joint détachable peut être détaché par voie électrolytique.
